# EUROPEAN PATENT APPLICATION

(11) **EP 4 596 035 A1**
(43) Date of publication of application: **06.08.2025**
(21) Application number: 24155765.1
(22) Date of filing: 05.02.2024
(51) Int. Cl.: A61N 5/06

(54) **LIGHTING TO PROVIDE PHOTOBIOMODULATION**

(71) Applicant: Sunled Life Science B.V., 1098XG Amsterdam (NL)
(72) Inventor: BERENDS, Anne, 1098 XG Amsterdam (NL); KRAMES, Michael, 1098 XG Amsterdam (NL); HILGERINK, Tom, 1098 XG Amsterdam (NL)
(74) Representative: Hoyng Rokh Monegier B.V.

(57) **Abstract**

Providing radiation for stimulating photobiomodulation in a user comprises providing radiation in the near-infrared spectrum with a beam angle of between 4-60° FAHP and continuous or pulsed with a duty cycle greater than 20%. When ambient light is detected or provided in the vicinity of the user at a brightness of greater than 10 nits, the radiation is generated and directed to illuminate the face and neck area of the user and/or to illuminate at least 350 cm² of the skin of the user, the radiation delivered to the skin of the user at an irradiance between 1 and 50 mW/cm² to provide to the user a dose of between 1 and 50 J/cm² within 1 hour and cumulative energy of between 1 and 30 kJ.

## Description

### TECHNICAL FIELD

The invention relates generally to a method and system for providing radiation to induce a photobiomodulation (PBM) response is a user.

### BACKGROUND ART

Photobiomodulation (PBM) involves irradiating a living organism at certain energy/power levels to induce biological or biochemical responses. The irradiation may be in the visible spectrum, such as red light, or in the non-visible spectrum, such as near-infrared (NIR). There has been a significant amount of research about the medical benefits of employing PBM therapy to provide physical and psychological benefits.

PBM may be induced in a user by exposing the user to suitable radiation over a long time period. This may be accomplished by including a suitable radiation source in electrical equipment such as overhead lighting fixtures, where it may be expected that the user is exposed to the radiation for several hours in a day. In this situation, it is beneficial to pulse the radiation source to avoid overdosing the user and reduce component count and driver power, while still providing sufficient irradiance to the user's skin to induce a PBM response in the user.

PBM may also be induced in a user by exposing the user to suitable radiation over a shorter time period, such as for an hour or less. In this situation, overdosing is generally not an issue and the radiation source may be energized continuously (i.e. not pulsed) or pulsed at a relatively high duty cycle to provide sufficient dose to the user in a shorter time. However, providing the radiation to a user in a safe and effective manner to achieve the desired benefits is difficult due to the large number of variables which should be controlled. Moreover, it is desirable to deliver the effective dose in most sustainable way: energy and cost effective.

Therefore, there is a need to overcome the abovementioned disadvantages of the currently available apparatuses and methods.

### SUMMARY OF THE INVENTION

It would be desirable to provide an apparatus that is easy to use, energy efficient, cost effective and yet emits an amount of radiation sufficient to induce PBM response.

In a first aspect, the invention provides a method for providing radiation for stimulating photobiomodulation in a user. The method comprises providing a radiation source for emitting radiation in the near-infrared (NIR) spectrum, wherein the radiation emitted by the radiation source has a beam angle of between 4-60° FAHP, and wherein the radiation is continuous or pulsed with a duty cycle greater than 20%. The method further comprises detecting if visible light in the vicinity of the user has a brightness of greater than 10 nits, or providing visible light in the vicinity of the user at a brightness of greater than 10 nits. The method further comprises, when visible light is detected or provided in the vicinity of the user at a brightness of greater than 10 nits: generating radiation from the radiation source, directing the radiation emitted by the radiation source to illuminate the face and neck area of the user and/or to illuminate at least 350 cm² of the skin of the user, and delivering the radiation to the skin of the user at an irradiance between 1 and 50 mW/cm² to provide to the user a dose of between 1 and 50 J/cm² within 1 hour and cumulative energy of between 1 and 30 kJ.

In another aspect, the invention provides an apparatus for providing radiation for stimulating photobiomodulation in a user. The apparatus comprises a radiation source configured to emit radiation in the near-infrared spectrum and at a beam angle of between 4-60° FAHP, and wherein the radiation source is configured to emit radiation that is continuous or pulsed with a duty cycle greater than 20%. The apparatus further comprises an light sensor configured to detect when visible light in the vicinity of the user has a brightness of greater than 10 nits, or a light source configured to provide visible light in the vicinity of the user at a brightness of greater than 10 nits. The apparatus is configured to: direct the radiation emitted by the radiation source to illuminate the face and neck area of the user and/or to illuminate at least 350 cm² of the skin of the user; and deliver the radiation to the user at an irradiance between 1 and 50 mW/cm² to provide a dose of between 1 and 50 J/cm² within 1 hour and cumulative energy of between 3 and 30 kJ.

### BRIEF DESCRIPTION OF THE DRAWINGS

Embodiments will now be described, by way of example only, with reference to the accompanying schematic drawings in which corresponding reference symbols indicate corresponding parts, and in which:
FIG. 1 is a schematic diagram of an apparatus for providing radiation for stimulating photobiomodulation in a user;
FIG. 2 is an illustration of a construction for accommodating a user and providing radiation for stimulating photobiomodulation in the user;
FIG. 3A is an illustration of an apparatus for providing radiation for stimulating photobiomodulation in the user integrated in a vanity mirror; and
FIG. 3B shows front and side views of another embodiment of the vanity mirror of FIG. 3A.

The figures are meant for illustrative purposes only, and do not serve as restriction of the scope or the protection as laid down by the claims.

### DESCRIPTION OF ILLUSTRATIVE EMBODIMENTS

The following is a description of certain embodiments of the invention, given by way of example only and with reference to the drawings.

A method and an apparatus for providing radiation for stimulating photobiomodulation in a user is disclosed. The method comprises providing a radiation source for emitting radiation in the near-infrared (NIR) spectrum, wherein the radiation emitted by the radiation source has a beam angle of between 4-60° FAHP (full-angle half power), and wherein the radiation is continuous or pulsed with a duty cycle greater than 20%. The method further comprises detecting if visible light in the vicinity of the user has a brightness of greater than 10 nits, or providing visible light in the vicinity of the user at a brightness of greater than 10 nits, and when visible light is detected or provided in the vicinity of the user at a brightness of greater than 10 nits, generating radiation from the radiation source, directing the radiation emitted by the radiation source to illuminate the face and neck area of the user and/or to illuminate at least 350 cm² of the skin of the user, and delivering the radiation to the skin of the user at an irradiance between 1 and 50 mW/cm² to provide to the user a dose of between 1 and 50 J/cm² within 1 hour and cumulative energy of between 1 and 30 kJ.

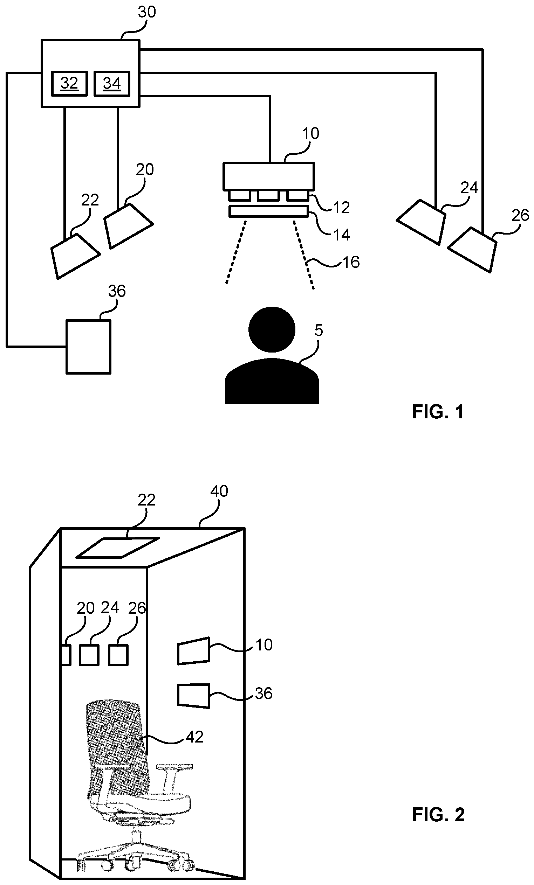

FIG. 1 is a schematic diagram of an apparatus 1 which may be used to perform the method. The apparatus 1 comprises a radiation source 10 configured to emit radiation in the near-infrared spectrum and at a beam angle of between 4-60° FAHP, wherein the radiation source is configured to emit radiation that is continuous or pulsed with a duty cycle greater than 20%.

The apparatus 1 further comprises a visible light sensor 20, or a visible light source 22, or both visible light sensor 20 and visible light source 22. The visible light sensor 20 is configured to detect when visible light in the vicinity of the user has a brightness of greater than 10 nits. This visible light may originate from light sources outside or separate from the apparatus 1 (e.g. ambient light from the sun or from lighting separate from the apparatus), or may originate at least in part from the visible light source 22. The visible light sensor 20 may be, e.g. a photodiode, a photoresistor, a phototransistor, or a photovoltaic light sensor.

The visible light source 22 is configured to provide visible light in the vicinity of the user 5 at a brightness of greater than 10 nits, which may be advantageous if there is not sufficient light from light sources outside or separate from the apparatus 1. The visible light source 22 may be, e.g. an LED or other light source providing visible light directed onto the user, e.g. white light.

The apparatus 1 is configured to direct the radiation emitted by the radiation source 10 to illuminate the face and neck area of the user 5 and/or to illuminate at least 350 cm² of the skin of the user 5, and deliver the radiation to the user 5 at an irradiance between 1 and 50 mW/cm² to provide a dose of between 1 and 50 J/cm² within 1 hour and cumulative energy of between 3 and 30 kJ.

The radiation source 10 may include one or more radiation emitting elements 12, which are preferably solid state devices such as LEDs or VCSELs. The radiation source is designed to emit the radiation in a narrow beam 16, having a beam angle of between 4° and 60° FAHP. This beam angle may be accomplished as a result of the design of the emitting elements 12 of the radiation source 10, or may be accomplished by using additional components 14 such as optical elements, e.g. lens, refractive, and/or reflective elements to achieve the desired beam angle (or both the design of the emitting elements 12 and additional components 14 may be used to achieve the beam angle).

The radiation may be continuous (i.e. non-pulsed, with a duty cycle of 100%) or pulsed with a duty cycle greater than 20% (where the duty cycle is the fraction of time the radiation source is energized and radiation is emitted during one pulse cycle). This enables a higher dose to be received by the user 5 over the exposure time compared to pulsing at lower duty cycles, so that shorter exposure times may nevertheless be effective to induce a PBM response in the user, e.g. one hour or less. The radiation source 10 may be pulsed such that a predetermined threshold irradiance is surpassed for a period of at least 1 ms during each pulse, wherein the predetermined threshold irradiance is 1 mW/cm² or more. A preferred way of achieving this pulse characteristic is with a squared wave form.

In an embodiment, the radiating elements are placed on a heatsink and active cooling might be applied to prevent overheating of the radiating elements.

The method of providing the radiation includes detecting if there is visible light in the vicinity of the user (e.g. using the visible light sensor 20) and/or providing visible light in the vicinity of the user (e,g. using the visible light source 22), and the radiation is preferably generated and delivered to the user only if the specified visible light is present. The presence of visible light leads to a constricted or less-dilated pupil, which limits the entry of the high intensity NIR light into the eye, and thus protects the retina from damage.

The radiation may be directed so that it illuminates the face and neck area of the user 5. The radiation may be specifically directed towards these areas of the user, for example so that more than 60% or more than 70% or more than 80% of the radiation illuminates the face and neck area of the user. Alternatively or additionally, the radiation may be directed so that it illuminates at least 350 cm² of the skin of the user, which may include the face and neck and other areas of the user or may be directed to or include areas such as the back or chest of the user.

The radiation is delivered to the surface (skin and eyes) of the user at an irradiance between 1 and 50 mW/cm². This threshold level of irradiance is necessary to induce a PBM response in the user. If the irradiance is too low it will not induce a PBM response, and too high irradiance may have undesirable health consequences. Irradiance is the power per unit area of electromagnetic radiation incident on a given surface, expressed here in milliwatts per square centimeter of irradiated surface. The irradiance delivered to the user is a function of the emitted power of the radiation source, the beam angle of the radiation, the duty cycle of the radiation, and the distance between the radiation source and the user. These variables are adjusted within the parameters of the disclosed method to achieve the required irradiance.

The radiation is delivered to the skin of the user to provide a dose of between 1 and 50 J/cm² to the user within one hour of exposure. This threshold level of dose is also necessary, in addition to the threshold level of irradiance, to induce a PBM response in the user. If the dosage is too low it will not induce a PBM response, and too high dosage may have undesirable health consequences. The dose received by the user is a function of irradiance delivered to the user and the period of exposure (according to the relation 1 J/cm² = 1 W/cm² . s). For example, an irradiance of 5 mW/cm² on the skin of the user for 30 min (=1800 s) would result in a dose of 1800 * 5 /1000 = 9 J/cm². A shorter exposure time may be used with a corresponding smaller dose received by the user, but a dose within the specified range should be received within a one hour exposure time.

In addition, the radiation is delivered to the user to provide cumulative energy of between 1 and 30 kJ to the user. The average projected surface area of a human face, neck, and hands and underarms (projected onto a plane in front of the user) corresponds to about 615 cm² (based on data published by NASA https://msis.jsc.nasa.gov/sections/section03.htm). An effective dose of 6.5 J/cm² thus corresponds to cumulative energy of 6.5 J/cm² * 615 cm² = 4 kJ imparted to the user.

In one embodiment, substantially all of the radiation emitted by the radiation source 10 is in the near-infrared (NIR) spectrum, for example from 750 nm to 1000 nm. For example, the radiation source 10 may be configured to emit greater than 95% of its radiation in the NIR spectrum. In another embodiment, substantially all of the radiation emitted by the radiation source 10 is in a spectrum covering the NIR and visible red light spectrums, for example from 620 nm to 1000 nm. For example, the radiation source 10 may be configured to emit in both the NIR and visible red spectrums, so that greater than 95% of its radiation is emitted in these two spectrums. For example, the radiation source 10 may comprise one or more radiation emitting elements 12 which emit in the NIR spectrum, e.g. between 750 nm and 1000 nm, and one or more radiation emitting elements 12 which emit in the visible red spectrum, e.g. between 620 nm and 750 nm.

The method preferably comprises detecting the presence of a user 5 within the working range of the radiation source 10, and deactivating the radiation source if the user is not detected within the working range. One embodiment comprises detecting when the user 5 is at a distance between 20 and 200 cm from the radiation source 10, and deactivating the radiation source 10 if the user 5 is not detected at a distance between 20 and 200 cm from the radiation source 10. Another embodiment comprises detecting when the user 5 is at a distance between 5 and 50 cm from the radiation source 10, and deactivating the radiation source 10 if the user 5 is not detected at a distance between 5 and 50 cm from the radiation source. This embodiment may be used, for example, when the radiation source is mounted in a shower or in or on or next to a mirror. Another embodiment comprises detecting when the user 5 is virtually in contact with the radiation source 10, e.g. at a distance between 0 and 5 cm from the radiation source 10, and deactivating the radiation source 10 if the user 5 is not detected at a distance larger than 5 cm from the radiation source 10. This embodiment may be used, for example, when the radiation source is mounted in or on a face mask or similar item worn by a user.

In an embodiment in which the user is virtually in contact with the radiation source 10, e.g. at a distance between 0 and 5 cm from the radiation source 10, it is advantageous that the radiation emitted by the radiation source has a beam angle >60° FAHP, for example 120°.

A proximity sensor 24 may be used to detect the presence of user 5 within the working range of radiation source 10, wherein the apparatus is configured to deactivate the radiation source if the user is not detected within the working range. The proximity sensor 24 may detect when the user 5 is at a required distance, and/or to detect the distance to the user 5. For example, an optical sensor (ToF sensor), an ultrasonic sensor, capacitive sensor, or other suitable type of sensor may be used. The proximity sensor 24 may generate a signal to indicate the presence of the user 5 at the relevant distance from the radiation source 10, the signal being used to activate the radiation source 10 to emit the radiation. The distance between the user 5 and the radiation source 10 may be calculated in various ways, e.g. based on the average distance or the minimum distance of the surface of the user to the radiation source 10 within the beam angle of the emitted radiation 16.

In one embodiment, the radiation source 10 is deactivated when a predetermined dose has been delivered to the user, wherein the predetermined dose may be between 1 and 50 J/cm² or between 1 and 30 kJ. The dose delivered to the user and/or the dosing time may be calculated for the user, and may be indicated to the user, e.g. via a user interface 36 (such as a display screen). The dosing time may be calculated based on a timer 32 which calculates the elapsed time when the radiation source is activated. The dose delivered to the user 5 may be calculated based on the dosing time and the detected distance between the user 5 and the radiation source 10, for example based on a signal from a proximity sensor 24 which detects the distance.

In one embodiment, a facial recognition sensor 26 is used to detect a user's face. The radiation source 10 may be activated when a user's face is detected in a predetermined position with respect to the radiation source. The facial recognition sensor 26 may be used to differentiate between users. In this way a dose amount delivered to each user 5 may be stored in a memory 34. A previously stored dose value for a user may be taken into account when calculating a subsequent radiation dose for that user, e.g. activating or deactivating the radiation source based at least in part on the stored dose. The previous stored dose and currently supplied dose may also be displayed to the user on the user interface 36.

The apparatus may include a control unit 30 which controls the radiation source 10. The control unit 30 may include the timer 32 and memory 34. The control unit 30 may receive inputs from the ambient light sensor 20, proximity sensor 24, and facial recognition sensor 26. The control unit 30 provide outputs for control of the radiation source 10, ambient light source 22, and user interface 36.

The apparatus described above with respect to FIG. 1 may be integrated in a construction configured to at least partially enclose the user. FIG. 2 illustrates an embodiment of such a construction 40, comprising a cubicle suitable for containing a user. For example, the construction 40 may comprise a cubicle, call cabin, relaxation pod, massage chair, shower (cabin), vanity mirror, toilet or similar. The construction 40 may be sufficiently large to accommodate more than one person and the apparatus 1 designed to provide radiation to multiple users at the same time.

The construction 40 includes the radiation source 10 (comprising one or more radiation emitting elements 12) mounted on a surface of the construction and positioned to direct the emitted radiation beam onto the user, for example onto the face and neck area of the user. For example, a chair 42 for seating the user may be placed in the construction 40 and the radiation source positioned to direct the emitted radiation beam onto the user when seated in the chair. This aids in reducing the variation in the positioning of the user (and the face and neck area of the user) within the construction 40.

The visible light source 22 is mounted to provide visible light to the interior of the construction, and the user interface 36 is positioned to be visible to the user. The visible light sensor 20, proximity sensor 24, and facial recognition sensor 26 are also mounted in the interior of the construction in suitable positions to sense the relevant parameter. The construction 40 preferably also houses the control unit 30 with timer 32 and memory 34.

A typical shower enclosure is about 90x90 cm. With NIR emitters integrated in the wall of the enclosure, the distance between the emitters and the user is <45 cm. For example, using ten SST-10-IRD-B50H-V850 LEDs with a Carclo 10413 10mm TIR optic, the resulting FAHP is 27.5° and the assumed LED+optic beam efficiency is 94.5%. With a CW driving mode and 1.45 A driving current, the average irradiance at the user's face is about 20.6 mW/cm² at 45 cm and corresponds with a dose of about 6.2 J/cm² delivered in 5 minutes. With an average projected area of 400 cm² (for face and neck), the cumulative dose is about 2.5 kJ. The proximity sensor 24 may be installed near the emitters to ensure irradiance does not exceed eye safety limits.

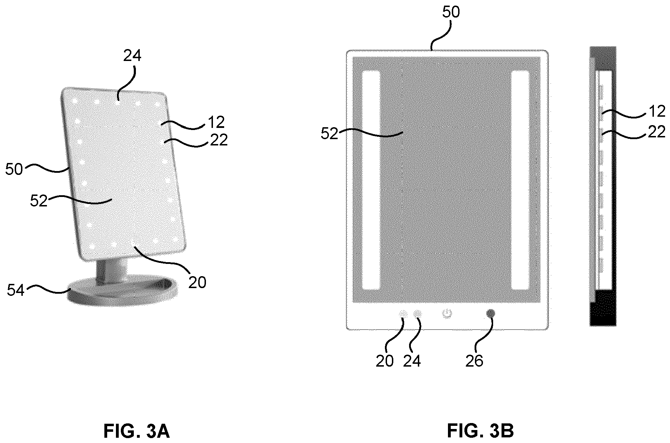

The apparatus described above with respect to FIG. 1 may be integrated into equipment, tools or objects to which the face and neck area of a user is commonly exposed. FIGs. 3A and 3B illustrate an embodiment of such an object in the form of a vanity mirror 50. The vanity mirror 50 includes the radiation source 10 comprising radiation emitting elements 12 mounted around the mirror surface facing the user, positioned to direct the emitted radiation beam onto the face and neck area of the user. The vanity mirror 50 is typically used at a distance of about 15 cm from a user. For example, when using three SST-10-IRD-B50H-V850 LEDs with a Carclo 10413 10mm TIR optic for the radiation emitting elements 12, a resulting FAHP of about 27.5° may be obtained and the assumed LED+optic beam efficiency is about 94.5%. With a CW (continuous wave) driving mode at 1.45 A driving current, the average irradiance at the user's face is about 14 mW/cm² and corresponds with a dose of approximately 12.6 J/cm². With an average projected area of about 400 cm² (for the face and neck of the user), the cumulative dose delivered is about 5.0 kJ.

A proximity sensor 24 may be installed in the mirror 50 to ensure irradiance does not exceed eye safety limits. The mirror 50 may include a visible light source 22 (for example a plurality of light emitters arranged on each side of the mirror surface 52) to cast light on the face and neck of the user. The mirror 50 may also include a visible light sensor 20, proximity sensor 24, and facial recognition sensor 26 as described above, and a control unit 30, e.g. mounted in the base 52 of the mirror 50.

For sustainability reasons, it is preferred that embodiments use as little material and as few components as possible. One way to achieve this is to combine functionalities by integrating PBM functionality into electronic devices that are already present or also serve another purpose. For integrated functionality, it is preferable that the PBM parts take up as little space as possible. Additionally, when in use, the embodiments preferably use as little energy as possible. This can be achieved by a combination of a) carefully defining the right surface area of the user to be irradiated, b) choosing the right beam angles for the NIR radiation, such that only the user space is irradiated with the NIR radiation, c) active dose monitoring, such that the NIR radiation source shuts off (or is reduced or modulated) when a predetermined dose has been delivered, d) using high-quality components such that electricity is converted into NIR radiation most efficiently (e.g. by using high-efficiency light sources such as LEDs or VCSELs), and e) that as much as possible of the emitted NIR radiation is folded into the desired beam angle (e.g. into a beam angle of between 4-60° FAHP) by use of primary and/or secondary optics).

Depending on the use case of the embodiments, e.g. user distance from the radiation source and space, user surface area to be irradiated, desired dosing time, desired dose, the most sustainable and economically feasible design can be created. In an embodiment, a dose of 4kJ is delivered to the face and neck of the user at an average distance of 75 cm with a total material use of <500 g, preferably <300 g. In another embodiment, e.g. the vanity mirror example above, the dose is delivered by using <300 g of materials (for the PBM functionality only).

The foregoing descriptions are intended to be illustrative, not limiting. It will be apparent to the person skilled in the art that alternative and equivalent embodiments of the invention can be conceived and reduced to practice, without departing from the scope of the invention described in the claims set out below.

## Claims

1. A method for providing radiation for stimulating photobiomodulation in a user, the method comprising:
providing a radiation source for emitting radiation in the near-infrared spectrum, wherein the radiation emitted by the radiation source has a beam angle of between 4-60° FAHP, and wherein the radiation is continuous or pulsed with a duty cycle greater than 20%;
detecting if visible light in the vicinity of the user has a brightness of greater than 10 nits, or providing visible light in the vicinity of the user at a brightness of greater than 10 nits; and
when visible light is detected or provided in the vicinity of the user at a brightness of greater than 10 nits:
generating radiation from the radiation source;
directing the radiation emitted by the radiation source to illuminate the face and neck area of the user and/or to illuminate at least 350 cm² of the skin of the user; and
delivering the radiation to the user at an irradiance between 1 and 50 mW/cm² to provide to the user a dose of between 1 and 50 J/cm² within 1 hour and cumulative energy of between 1 and 30 kJ.

2. The method of claim 1, wherein substantially all of the radiation emitted by the radiation source is in the near-infrared spectrum.

3. The method of claim 1, wherein substantially all of the radiation emitted by the radiation source is in the near-infrared spectrum or in the visible red spectrum.

4. The method of any one of the preceding claims, further comprising detecting presence of a user within a working range of the radiation source, and deactivating the radiation source if the user is not detected within the working range.

5. The method of any one of the preceding claims, further comprising deactivating the radiation source when a predetermined dose has been delivered to the user, wherein the predetermined dose is between 1 and 50 J/cm².

6. The method of any one of the preceding claims, further comprising using a facial recognition sensor to detect a user's face, and activating the radiation source when the user's face is detected in a predetermined position with respect to the radiation source.

7. An apparatus for providing radiation for stimulating photobiomodulation in a user, the apparatus comprising:
a radiation source configured to emit radiation in the near-infrared spectrum and at a beam angle of between 4-60° FAHP, and wherein the radiation source is configured to emit radiation that is continuous or pulsed with a duty cycle greater than 20%;
a visible light sensor configured to detect when visible light in the vicinity of the user has a brightness of greater than 10 nits, or a light source configured to provide visible light in the vicinity of the user at a brightness of greater than 10 nits; and
wherein the apparatus is configured to:
direct the radiation emitted by the radiation source to illuminate the face and neck area of the user and/or to illuminate at least 350 cm² of the skin of the user; and
deliver the radiation to the user at an irradiance between 1 and 50 mW/cm² to provide a dose of between 1 and 50 J/cm² within 1 hour and cumulative energy of between 3 and 30 kJ.

8. The apparatus of claim 7, wherein the radiation source is configured to emit substantially all of the radiation in the near-infrared spectrum.

9. The apparatus of claim 7, wherein the radiation source is configured to emit substantially all of the radiation in the near-infrared spectrum and/or the visible red spectrum.

10. The apparatus of any one of claims 7-9, further comprising a proximity sensor configured to detect presence of a user within a working range of the radiation source, wherein the apparatus is configured to deactivate the radiation source if the user is not detected within the working range.

11. The apparatus of any one of claims 7-10, wherein the apparatus is configured to deactivate the radiation source when a predetermined dose has been delivered to the user, wherein the predetermined dose is between 1 and 50 J/cm².

12. The apparatus of any one of claims 7-11, wherein the apparatus comprises a facial recognition sensor configured to detect a user's face, and wherein the apparatus is configured to activate the radiation source when the user's face is detected in a predetermined position with respect to the radiation source.

13. The apparatus of any one of claims 7-12, wherein the apparatus comprises a facial recognition sensor configured to detect a user's face, and wherein the apparatus is configured to store a dose delivered by the apparatus to the user over a predetermined period.

14. The apparatus of any one of claims 7-13, wherein the apparatus is integrated in a construction configured to at least partially enclose the user

15. The apparatus of any one of the claims 7-14, wherein the radiation source is pulsed such that a predetermined threshold irradiance is surpassed for a period of at least 1 ms during each pulse, wherein the predetermined threshold irradiance is 1 mW/cm² or more.

16. The apparatus of any one of the claims 7-14, wherein the apparatus is configured to deactivate the radiation source when the user is too close to the radiation source to prevent retinal damage and/or to adhere to eye safety standards.
